# EUROPEAN PATENT APPLICATION

(11) **EP 4 273 214 A1**
(43) Date of publication of application: **08.11.2023**
(21) Application number: 22749601.5
(22) Date of filing: 27.01.2022
(51) Int. Cl.: C12M 1/26, C12N 5/00, C12N 1/00, C12Q 1/24, G01N 1/00

(54) **SAMPLING METHOD**

(30) Priority: 02.02.2021 JP 2021014846
(71) Applicant: TERUMO Kabushiki Kaisha, Tokyo 151-0072 (JP)
(72) Inventor: IGARASHI, Masatsugu, Ashigarakami-gun, Kanagawa 259-0151 (JP)
(74) Representative: TBK
(86) International application number: PCT/JP2022/003051
(87) International publication number: WO 2022/168727

(57) **Abstract**

A sampling method includes: a sampling step of circulating a sample from a cell culture device (200) to a sampling channel (30) and measuring concentrations of predetermined components in the sample by a second sensor (28) and a gas concentration sensor (62); a cleaning step of circulating a cleaning liquid through the sampling channel (30) to circulate the cleaning liquid through the second sensor (28) and the gas concentration sensor (62) after the sampling step; and an air introduction step of replacing the cleaning liquid remaining in the gas concentration sensor (62) with air after the cleaning step.

## Description

### Technical Field

The present invention relates to a sampling method.

### Background Art

For example, U.S. Patent No. 9442047 discloses a sampling method using a sampling system including a sampling channel that collects a liquid sample from a cell culture device.

### Summary of Invention

By the way, a sampling system may include a biosensor and a gas concentration sensor provided in a sampling channel so as to be in contact with a sample, and an introduction path that introduces a cleaning liquid to an upstream side of the biosensor and the gas concentration sensor in the sampling channel. In this case, for example, a sampling step of circulating the sample in the sampling channel to bring the sample into contact with the biosensor and the gas concentration sensor, and a cleaning step of introducing the cleaning liquid from the introduction path into the sampling channel to remove the sample attached to the biosensor in order to prevent deterioration of a catalyst of the biosensor are performed.

When the cleaning step is completed, the cleaning liquid remains inside the gas concentration sensor. In a case where the capacity inside the gas concentration sensor is relatively large, the cleaning liquid may be mixed into the sample inside the gas concentration sensor during the sampling step. This decreases measurement accuracy of the gas concentration sensor.

The present invention has been made in view of such problems, and an object of the present invention is to provide a sampling method capable of removing a sample attached to a biosensor and preventing decrease in measurement accuracy of a gas concentration sensor.

One aspect of the present invention is a sampling method using a sampling system including a sampling channel that collects a liquid sample of a cell culture device, a biosensor and a gas concentration sensor being provided in the sampling channel so as to be in contact with the sample, the sampling method including: a sampling step of circulating the sample from the cell culture device to the sampling channel and measuring concentrations of predetermined components in the sample by the biosensor and the gas concentration sensor; a cleaning step of circulating a cleaning liquid through the sampling channel to circulate the cleaning liquid through the biosensor and the gas concentration sensor after the sampling step; and an air introduction step of replacing the cleaning liquid remaining in the gas concentration sensor with air after the cleaning step, in which the sampling step is performed two or more times, and the second and subsequent sampling steps are performed after the air introduction step.

According to the present invention, the cleaning step is performed after the sampling step, so that the sample attached to the biosensor during the sampling step can be removed by the cleaning liquid in the cleaning step. In the air introduction step after the cleaning step, the cleaning liquid remaining in the gas concentration sensor is replaced with air. It is therefore possible to prevent the cleaning liquid from being mixed into the sample inside the gas concentration sensor in the sampling step after the cleaning step. It is therefore possible to prevent decrease in measurement accuracy of the gas concentration sensor.

### Brief Description of Drawings

Fig. 1 is a schematic configuration diagram of a sampling system according to an embodiment of the present invention.
Fig. 2 is a configuration explanatory diagram of a main part of a cell culture device.
Fig. 3 is a flowchart for explaining a sampling method using the sampling system of Fig. 1.
Fig. 4 is a flowchart for explaining a sampling step in Fig. 3.
Fig. 5 is a first operation explanatory diagram of the sampling method.
Fig. 6 is a second operation explanatory diagram of the sampling method.
Fig. 7 is a third operation explanatory diagram of the sampling method.
Fig. 8 is a fourth operation explanatory diagram of the sampling method.
Fig. 9 is a fifth operation explanatory diagram of the sampling method.

### Description of Embodiment

Hereinafter, a preferred embodiment of a sampling method according to the present invention will be described with reference to the accompanying drawings.

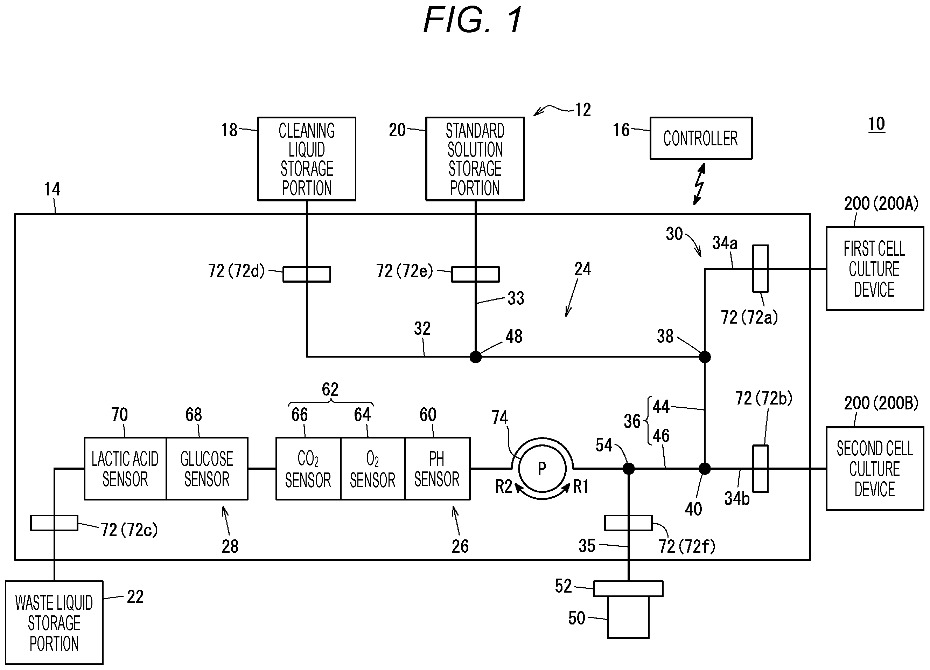

As illustrated in Fig. 1, a sampling system 10 according to an embodiment of the present invention collects liquid samples of a plurality of cell culture devices 200 and measures concentrations of predetermined components in the samples. The sampling system 10 includes a sampling kit 12, a circuit control device 14 to which the sampling kit 12 is detachable, and a controller 16. The sampling kit 12 is a disposable product, and the circuit control device 14 is a reusable product that can be reused.

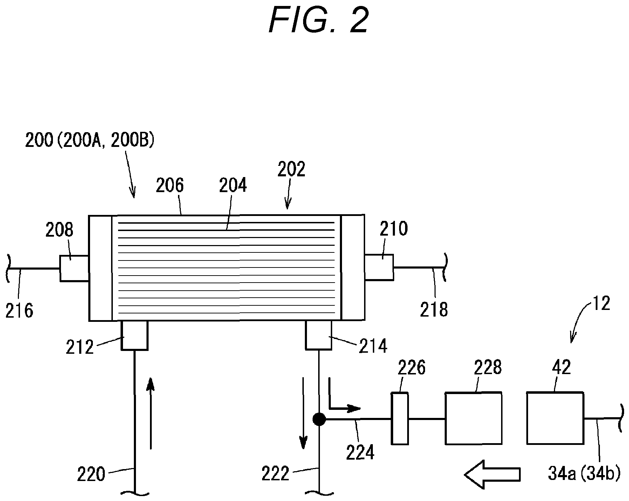

In the present embodiment, as the plurality of cell culture devices 200, a first cell culture device 200A and a second cell culture device 200B are connected to the sampling kit 12. As illustrated in Figure 2, the cell culture device 200 has a bioreactor 202 for culturing cells. The cells to be cultured are separated from a biological tissue, and for example, cells (T cells, and the like), and stem cells (ES cells, iPS cells, mesenchymal stem cells, etc.) contained in blood are used.

The bioreactor 202 is configured as a so-called hollow fiber bioreactor. The bioreactor 202 includes a number (plurality) of hollow fibers 204 and a cylindrical housing 206 storing these hollow fibers 204. A wall portion constituting the hollow fiber 204 is formed with a plurality of pores (not illustrated). Through the pores, an intra capillary (IC) region which is a lumen of the hollow fiber 204 is communicated with an extra capillary (EC) region located outside the hollow fiber 204 in the housing 206. A diameter of the pores is set to a size that allows passage of a low molecular weight (for example, water, ions, oxygen, lactate, and the like) while blocking passage of a high molecular weight (cell, or the like).

The housing 206 is provided with an IC inlet port 208, an IC outlet port 210, an EC inlet port 212, and an EC outlet port 214. The IC inlet port 208 is provided at one end of the housing 206. The IC inlet port 208 introduces a liquid (a solution containing cells, a medium, or the like) guided from an IC inlet flow path 216 into the IC region of the bioreactor 202. The IC outlet port 210 is provided at the other end of the housing 206. The IC outlet port 210 directs a liquid flowing through the IC region of the bioreactor 202 to an IC outlet flow path 218.

The EC inlet port 212 and the EC outlet port 214 are provided on an outer peripheral surface of the housing 206. The EC inlet port 212 introduces a medium directed from an EC inlet flow path 220 into the EC region of the bioreactor 202. The EC outlet port 214 directs a medium flowing through the EC region of the bioreactor 202 to an EC outlet flow path 222. As the medium, an appropriate medium may be selected according to cells of a living body, and for example, a medium prepared by adding various amino acids, vitamins, serum, and the like, using a balanced salt solution (BSS) as a basic solution is used.

A connection line 224 for guiding the culture medium circulating in the EC region to the sampling kit 12 is connected to the EC outlet flow path 222. The connection line 224 is provided with a sterile filter 226 and a sampling connector 228. The sterile filter 226 aseptically holds a portion of the cell culture device 200 closer to the EC outlet flow path 222 than the sterile filter 226. An introduction connector 42 of the sampling kit 12 is detachable from the sampling connector 228.

In the present embodiment, the sampling system 10 collects, as a sample, a culture medium circulating in the EC region of the cell culture device 200. However, the sample collected by the sampling system 10 is not limited to the medium circulating in the EC region and may be a medium or another liquid circulating in the IC region.

In Fig. 1, the sampling kit 12 includes a cleaning liquid storage portion 18, a standard solution storage portion 20, a waste liquid storage portion 22, a connection circuit 24, a first sensor 26, and a second sensor 28.

The cleaning liquid storage portion 18, the standard solution storage portion 20, and the waste liquid storage portion 22 are formed in a bag shape by using a flexible material made of a soft resin such as polyvinyl chloride or polyolefin, for example. However, the cleaning liquid storage portion 18, the standard solution storage portion 20, and the waste liquid storage portion 22 can be appropriately changed as long as they can store liquid.

The cleaning liquid storage portion 18 stores a cleaning liquid. As the cleaning liquid, a buffer solution or physiological saline is used. Examples of the buffer solution include phosphate buffered saline (PBS) and tris-buffered saline (TBS). However, the cleaning liquid is not limited to those described above.

The standard solution storage portion 20 stores a standard solution. The standard solution is a liquid for calibrating the first sensor 26 and the second sensor 28. Specifically, the standard solution is a liquid in which a PH value, an O₂ value (oxygen concentration), a CO₂ value (carbon dioxide concentration), a glucose value (glucose concentration), and a lactic acid value (lactic acid concentration) are set to prescribed values.

The waste liquid storage portion 22 stores a waste liquid (a sample, a cleaning liquid, and a standard solution) circulating in the connection circuit 24. The waste liquid storage portion 22 is an empty bag in which no liquid is stored before the sampling kit 12 is used.

The connection circuit 24 includes a sampling channel 30 that collects a sample of a cell culture device 200, an introduction path 32 that guides a cleaning liquid to the sampling channel 30, a standard solution introduction path 33 that guides a standard solution to the introduction path 32, and an air introduction path 35 that guides air to the sampling channel 30. The sampling channel 30 includes a first sample introduction path 34a, a second sample introduction path 34b, and a sample flow path 36.

The first sample introduction path 34a guides the sample (culture medium) of the first cell culture device 200A to the sample flow path 36. The introduction connector 42 attached to the sampling connector 228 of the first cell culture device 200A is provided at one end of the first sample introduction path 34a (see Fig. 2). The other end of the first sample introduction path 34a is connected to one end of the sample flow path 36. Hereinafter, a connection portion between the first sample introduction path 34a and the sample flow path 36 is referred to as a first connection portion 38.

The second sample introduction path 34b guides the sample (culture medium) of the second cell culture device 200B to the sample flow path 36. The introduction connector 42 attached to the sampling connector 228 of the second cell culture device 200B is provided at one end of the second sample introduction path 34b (see Fig. 2). The other end of the second sample introduction path 34b is connected to an intermediate portion of the sample flow path 36. Hereinafter, a connection portion between the second sample introduction path 34b and the sample flow path 36 is referred to as a second connection portion 40.

The sample flow path 36 includes an intermediate flow path 44 that connects the first connection portion 38 and the second connection portion 40 to each other, and a sensor flow path 46 that connects the second connection portion 40 and the waste liquid storage portion 22 to each other.

One end of the introduction path 32 is connected to the cleaning liquid storage portion 18. The other end of the introduction path 32 is connected to the first connection portion 38. One end of the standard solution introduction path 33 is connected to the standard solution storage portion 20. The other end of the standard solution introduction path 33 is connected to an intermediate portion of the introduction path 32. Hereinafter, a connection portion between the introduction path 32 and the standard solution introduction path 33 is referred to as a third connection portion 48.

An air port portion 50 opened to the atmosphere and a sterile filter 52 are provided at one end of an air introduction path 35. The sterile filter 52 holds the connection circuit 24 in a sterile state. The other end of the air introduction path 35 is connected to a portion between the second connection portion 40 and the first sensor 26 in the sensor flow path 46. Hereinafter, the connection portion between the sensor flow path 46 and the air introduction path 35 is referred to as a fourth connection portion 54.

A first sensor 26 and a second sensor 28 are provided in the sensor flow path 46 so as to be in contact with the sample. The first sensor 26 is an integrally molded product and includes a PH sensor 60 and a gas concentration sensor 62. The PH sensor 60 measures PH in the sample. The gas concentration sensor 62 measures a gas concentration in the sample. Specifically, the gas concentration sensor 62 includes an O₂ sensor 64 that measures an O₂ concentration in the sample and a CO₂ sensor 66 that measures a CO₂ concentration in the sample.

The second sensor 28 is, for example, a biosensor such as an enzyme sensor. The second sensor 28 is provided downstream of the first sensor 26 in the sensor flow path 46. The second sensor 28 is an integrally molded product and includes a glucose sensor 68 that measures a glucose concentration in the sample and a lactic acid sensor 70 that measures a lactic acid concentration in the sample. The second sensor 28 is not limited to the enzyme sensor and may include a non-enzyme type glucose sensor. The measurement items of the second sensor 28 are not limited to glucose and lactic acid, and may include glutamic acid, or the like.

The circuit control device 14 includes a plurality of clamps 72 and one pump 74. In the present embodiment, the circuit control device 14 includes, as the plurality of clamps 72, a first clamp 72a (first opening/closing portion), a second clamp 72b, a third clamp 72c, a fourth clamp 72d (second opening/closing portion), a fifth clamp 72e, and a sixth clamp 72f (third opening/closing portion).

The first clamp 72a is disposed so as to face the first sample introduction path 34a in a state where the sampling kit 12 is attached to the circuit control device 14 (hereinafter, referred to as a "set state") and opens and closes an internal flow path of the first sample introduction path 34a. The second clamp 72b is disposed so as to face the second sample introduction path 34b in the set state and opens and closes an internal flow path of the second sample introduction path 34b. The third clamp 72c is disposed so as to face a portion of the sensor flow path 46 between the second sensor 28 and the waste liquid storage portion 22 in the set state and opens and closes an internal flow path of the portion of the sensor flow path 46. The fourth clamp 72d is disposed so as to face a portion of the introduction path 32 on the upstream side of the third connection portion 48 in the set state and opens and closes an internal flow path of the portion of the introduction path 32. The fifth clamp 72e is disposed so as to face the standard solution introduction path 33 in the set state and opens and closes an internal flow path of the standard solution introduction path 33. The sixth clamp 72f is disposed so as to face the air introduction path 35 in the set state and opens and closes an internal flow path of the air introduction path 35.

The pump 74 rotates so as to strip off a wall portion constituting a flow path (tube) of the connection circuit 24, thereby applying a flow force to the liquid inside. The pump 74 is disposed so as to be in contact with a portion of the sensor flow path 46 between the second connection portion 40 and the first sensor 26 in a set state. The pump 74 performs first rotation operation (rotation operation in a direction of an arrow R1) such that a flow force in a direction toward the first sensor 26 (waste liquid storage portion 22) is applied to the liquid circulating through the sensor flow path 46. The pump 74 may perform second rotational operation (rotational operation in a direction of an arrow R2) such that a flow force in the direction toward the second connection portion 40 is applied to the liquid circulating through the sensor flow path 46.

The controller 16 (control unit) is a computer having a processor, a memory, and an input/output interface (not illustrated). The controller 16 performs overall control of the entire system by the processor executing a program stored in the memory. The controller 16 is connected to the circuit control device 14 by communication means including wired, wireless, network, or a combination thereof. Specifically, the controller 16 controls operation of the plurality of clamps 72 and the pump 74.

Next, a sampling method using the sampling system 10 will be described.

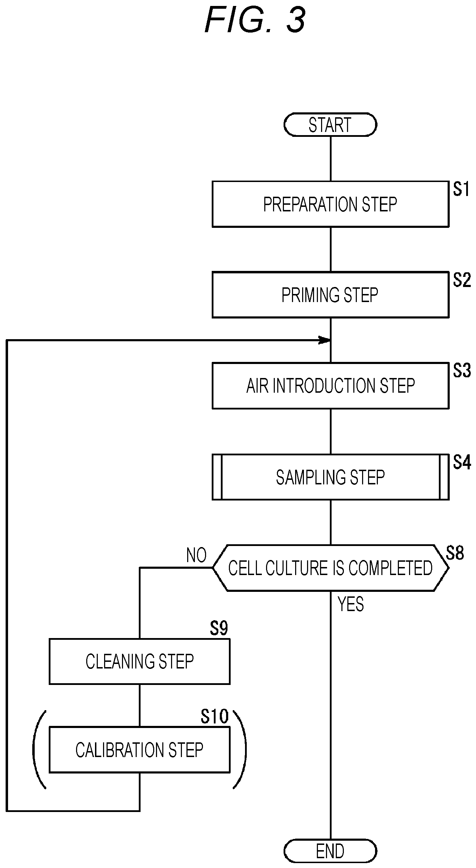

As illustrated in Fig. 3, the sampling method includes a preparation step, a priming step, an air introduction step, a sampling step, a cleaning step, and a calibration step.

First, in the preparation step (step S1), as illustrated in Figs. 1 and 2, the sampling kit 12 is attached (set) to the circuit control device 14, the introduction connector 42 of the first sample introduction path 34a is connected to the sampling connector 228 of the first cell culture device 200A, and the introduction connector 42 of the second sample introduction path 34b is connected to the sampling connector 228 of the second cell culture device 200B.

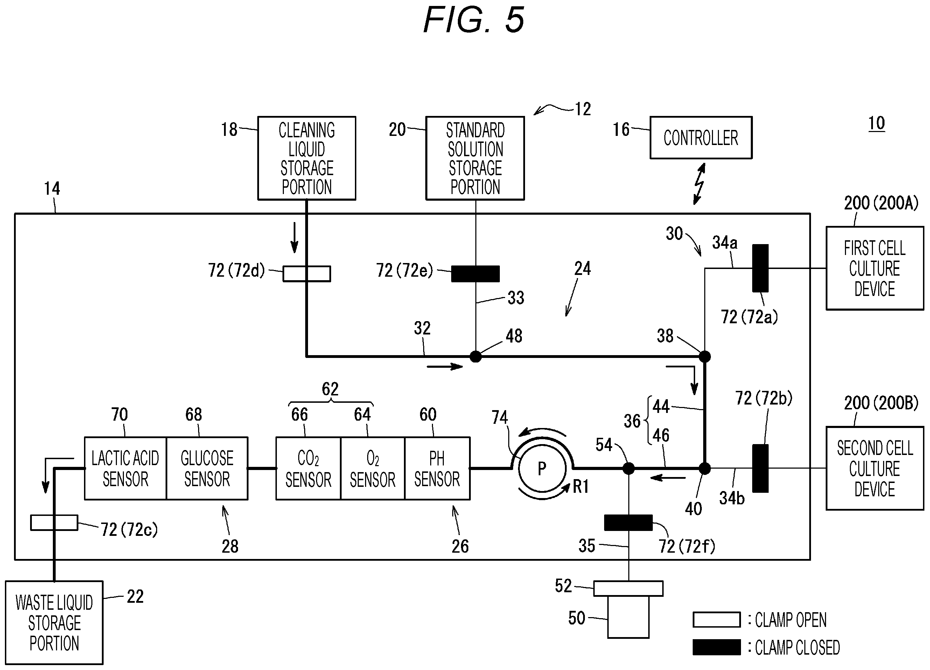

Subsequently, in the priming step (step S2 in Fig. 3), as illustrated in Fig. 5, the controller 16 causes the pump 74 to perform the first rotation operation in a state where the third clamp 72c and the fourth clamp 72d are opened and the first clamp 72a, the second clamp 72b, the fifth clamp 72e, and the sixth clamp 72f are closed. Then, the cleaning liquid in the cleaning liquid storage portion 18 is guided from the introduction path 32 to the waste liquid storage portion 22 via the first connection portion 38, the intermediate flow path 44, the second connection portion 40, and the sensor flow path 46 by the action of the pump 74.

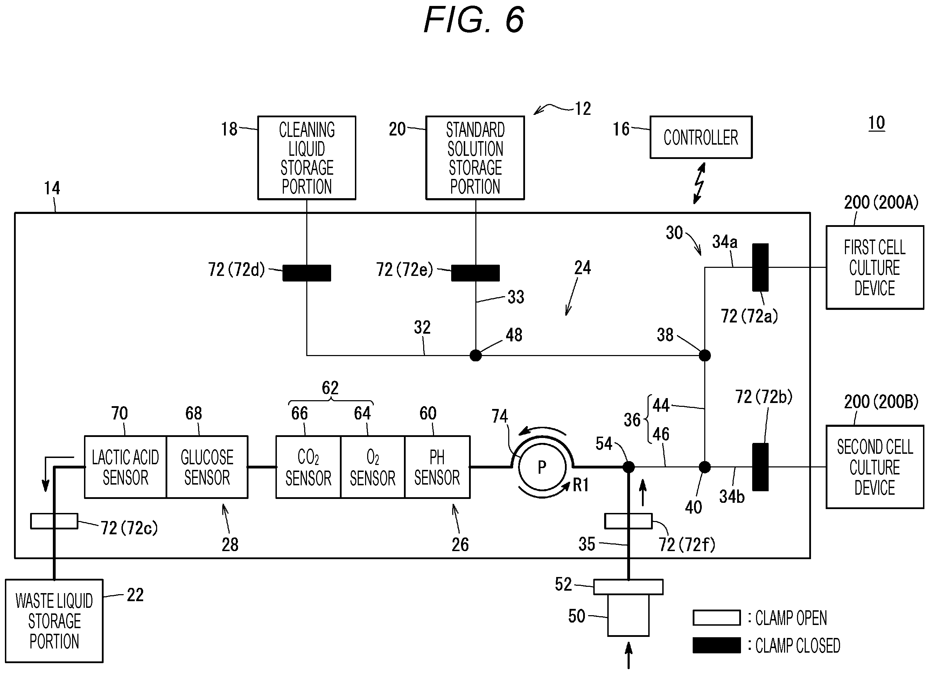

Thereafter, the air introduction step (step S3 in Fig. 3) is performed. Specifically, as illustrated in Fig. 6, the controller 16 causes the pump 74 to perform the first rotation operation in a state where the third clamp 72c and the sixth clamp 72f are opened and the first clamp 72a, the second clamp 72b, the fourth clamp 72d, and the fifth clamp 72e are closed. Then, the air introduced from the air port portion 50 to the air introduction path 35 via the sterile filter 52 is guided to the waste liquid storage portion 22 via the first sensor 26 and the second sensor 28 of the sensor flow path 46. As a result, the cleaning liquid remaining in the first sensor 26 is pushed out to the waste liquid storage portion 22.

The air introduction step is not limited to an example where the air introduced into the air introduction path 35 is guided to the waste liquid storage portion 22. In the air introduction step, it is sufficient that the cleaning liquid remaining in the gas concentration sensor 62 is replaced with air. In other words, a timing of stopping introduction of air (stopping driving of the pump 74) in the air introduction step may be a time point at which the air is guided to the portion between the first sensor 26 and the second sensor 28 of the sensor flow path 46.

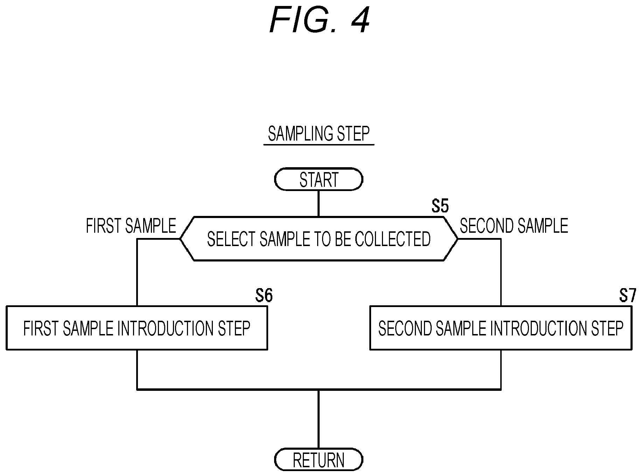

Then, the sampling step (step S4 in Fig. 3) is performed. Specifically, the controller 16 selects a sample to be collected (step S5 in Fig. 4). In other words, the controller 16 selects which one of the sample (first sample) of the first cell culture device 200A and the sample (second sample) of the second cell culture device 200B is to be collected on the basis of a cell culture state of the cell culture device 200.

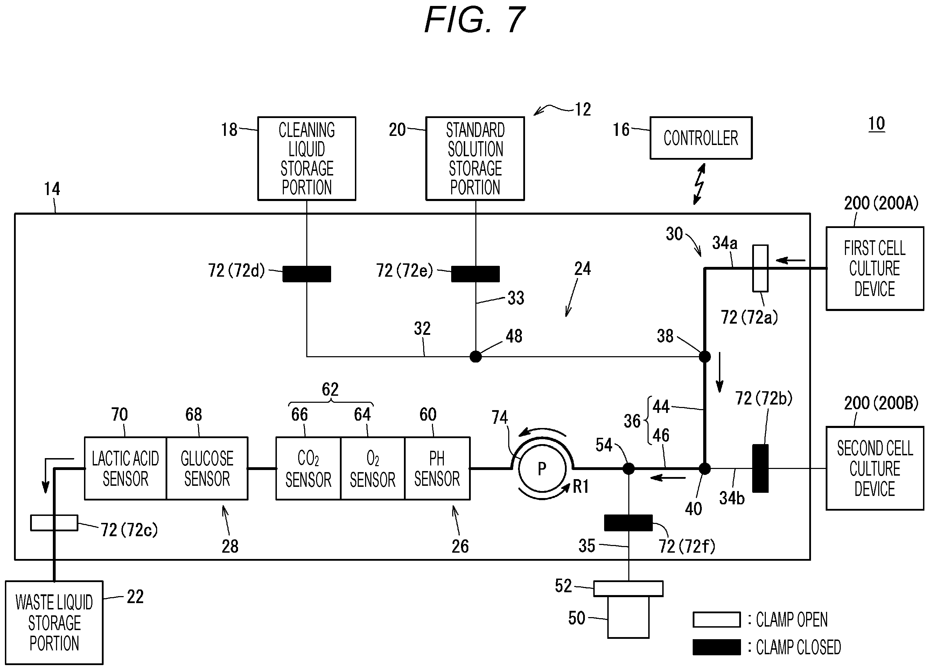

In a case where the controller 16 selects to collect the first sample, the first sample introduction step is performed (step S6 in Fig. 4). In other words, as illustrated in Fig. 7, the controller 16 causes the pump 74 to perform the first rotation operation in a state where the first clamp 72a and the third clamp 72c are opened and the second clamp 72b, the fourth clamp 72d, the fifth clamp 72e, and the sixth clamp 72f are closed. Then, the first sample of the first cell culture device 200A is guided to the waste liquid storage portion 22 via the first sample introduction path 34a, the first connection portion 38, the intermediate flow path 44, the second connection portion 40, and the sensor flow path 46 by the action of the pump 74.

In this event, the cleaning liquid does not remain in the first sensor 26 (the cleaning liquid in the first sensor 26 is replaced with air in the air introduction step), and thus, the cleaning liquid is not mixed into the first sample circulating through the first sensor 26.

The first sensor 26 and the second sensor 28 are in contact with the first sample. In the first sensor 26, the PH, the O₂ concentration, and the CO₂ concentration in the first sample are measured. The measurement results of the first sensor 26 are transmitted to the controller 16. In the second sensor 28, the glucose concentration and the lactic acid concentration in the first sample are measured. The measurement results of the second sensor 28 are transmitted to the controller 16. The controller 16 controls culture conditions of the first cell culture device 200A on the basis of the measurement results of the first sensor 26 and the second sensor 28.

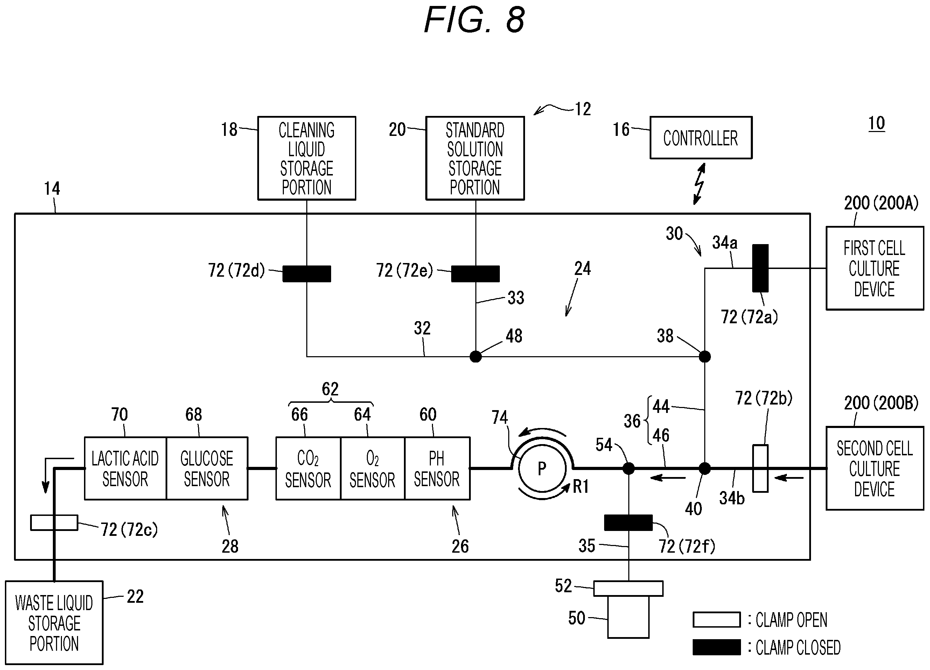

In a case where the controller 16 selects to collect the second sample, the second sample introduction step is performed (step S7 in Fig. 4). In other words, as illustrated in Fig. 8, the controller 16 causes the pump 74 to perform the first rotation operation in a state where the second clamp 72b and the third clamp 72c are opened and the first clamp 72a, the fourth clamp 72d, the fifth clamp 72e, and the sixth clamp 72f are closed. Then, the second sample of the second cell culture device 200B is guided to the waste liquid storage portion 22 via the second sample introduction path 34b, the second connection portion 40, and the sensor flow path 46 by the action of the pump 74.

In this event, the cleaning liquid does not remain in the first sensor 26 (the cleaning liquid in the first sensor 26 is replaced with air in the air introduction step), and thus, the cleaning liquid is not mixed into the second sample circulating through the first sensor 26.

The first sensor 26 and the second sensor 28 are in contact with the second sample. In the first sensor 26, the PH, the O₂ concentration, and the CO₂ concentration in the second sample are measured. The measurement results of the first sensor 26 are transmitted to the controller 16. In the second sensor 28, the glucose concentration and the lactic acid concentration in the second sample are measured. The measurement results of the second sensor 28 are transmitted to the controller 16. The controller 16 controls culture conditions of the second cell culture device 200B on the basis of the measurement results of the first sensor 26 and the second sensor 28.

Upon completion of the sampling step, the controller 16 in Fig. 3 determines whether or not the cell culture of the first cell culture device 200A and the second cell culture device 200B has been completed (step S8). In a case where the controller 16 determines that the cell culture has not been completed (step S8: NO), the cleaning step (step S9) is performed. In the cleaning step, as illustrated in Fig. 5, the controller 16 operates the plurality of clamps 72 and the pump 74 similarly to the priming step. Then, the cleaning liquid in the cleaning liquid storage portion 18 circulates in the first sensor 26 and the second sensor 28 and is guided to the waste liquid storage portion 22.

As a result, in the first sensor 26, the sample attached to each of the PH sensor 60, the O₂ sensor 64, and the CO₂ sensor 66 is removed by the cleaning liquid. In the second sensor 28, the sample attached to each of the glucose sensor 68 and the lactic acid sensor 70 is removed by the cleaning liquid.

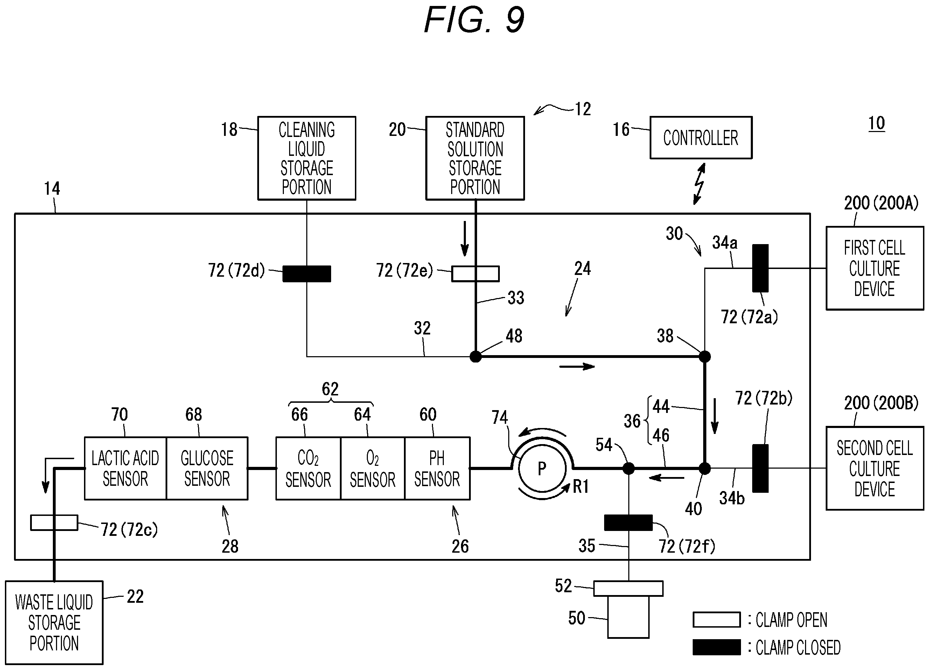

Thereafter, the calibration step (step S10 in Fig. 3) is performed as necessary. In the calibration step, as illustrated in Fig. 9, the controller 16 opens the third clamp 72c and the fifth clamp 72e and causes the pump 74 to perform the first rotation operation in a state where the first clamp 72a, the second clamp 72b, the fourth clamp 72d, and the sixth clamp 72f are closed. Then, the standard solution in the standard solution storage portion 20 is guided to the waste liquid storage portion 22 via the standard solution introduction path 33, the introduction path 32, the first connection portion 38, the intermediate flow path 44, the second connection portion 40, and the sensor flow path 46 by the action of the pump 74.

In this event, the first sensor 26 measures the PH, the O₂ concentration, and the CO₂ concentration in the standard solution. The measurement results of the first sensor 26 are transmitted to the controller 16. The controller 16 calibrates the PH sensor 60, the O₂ sensor 64, and the CO₂ sensor 66 on the basis of the measurement results of the first sensor 26. The second sensor 28 measures the glucose concentration and the lactic acid concentration in the standard solution. The measurement results of the second sensor 28 are transmitted to the controller 16. The controller 16 calibrates the glucose sensor 68 and the lactic acid sensor 70 on the basis of the measurement results of the second sensor 28. When the calibration step is completed, the steps after step S3 are sequentially performed. In the present embodiment, the sampling step is performed two or more times.

In a case where it is determined by the controller 16 that the cell culture has been completed (step S8 in Fig. 3: YES), a series of operation flow ends.

The present embodiment has the following effects.

According to the present embodiment, the cleaning step is performed after the sampling step, and thus, the sample attached to the second sensor 28 during the sampling step can be removed by the cleaning liquid in the cleaning step. In the air introduction step after the cleaning step, the cleaning liquid remaining in the gas concentration sensor 62 is replaced with air. It is therefore possible to prevent the cleaning liquid from being mixed into the sample inside the gas concentration sensor 62 in the sampling step after the cleaning step. It is therefore possible to prevent decrease in measurement accuracy of the gas concentration sensor 62.

The sampling system 10 includes an introduction path 32 that introduces a cleaning liquid into the sampling channel 30 to an upstream side of the second sensor 28, an air introduction path 35 connected to the sampling channel 30 to an upstream side of the gas concentration sensor 62 and opened to the atmosphere, a first clamp 72a that opens and closes the sampling channel 30 to an upstream side of a first connection portion 38 with the introduction path 32, a fourth clamp 72d that opens and closes the introduction path 32, a sixth clamp 72f that opens and closes the air introduction path 35, and a pump 74 provided in the sampling channel 30. In the air introduction step, air is guided from the air introduction path 35 to the sampling channel 30 by driving the pump 74 in a state where the first clamp 72a and the fourth clamp 72d are closed and the sixth clamp 72f is opened.

According to such a method, air can be introduced from the air introduction path 35 to the gas concentration sensor 62 with a simple configuration and control.

The air introduction path 35 is provided with the sterile filter 52. In the air introduction step, the air that has passed through the sterile filter 52 is guided to the sampling channel 30.

According to such a method, the sample can be held in a sterile state by the sterile filter 52.

The sampling channel 30 includes a sample flow path 36 provided with a second sensor 28 and a gas concentration sensor 62 and forming a downstream side of the first connection portion 38 with the introduction path 32 in the sampling channel 30, a first sample introduction path 34a that guides the first sample of the first cell culture device 200A to the first connection portion 38, and a second sample introduction path 34b that guides the second sample of the second cell culture device 200B to an upstream side of the second sensor 28 and the gas concentration sensor 62 in the sample flow path 36. The sampling step includes a first sample introduction step of introducing the first sample of the first cell culture device 200A from the first sample introduction path 34a into the sample flow path 36, and a second sample introduction step of introducing the second sample of the second cell culture device 200B from the second sample introduction path 34b into the sample flow path 36.

According to such a method, it is possible to prevent the cleaning liquid from being mixed into the first sample or the second sample inside the gas concentration sensor 62.

The sampling system 10 may collect a sample of one cell culture device 200 and measure concentrations of predetermined components. In this case, the sampling system 10 does not have to include the second sample introduction path 34b. In addition, the sampling system 10 may collect samples of three or more cell culture devices 200 individually and measure concentrations of predetermined components. In other words, the number of cell culture devices 200 connected to the sampling channel 30 may be three or more. In this case, sample introduction paths of the number corresponding to the number of cell culture devices 200 are provided. The air introduction path 35 may be connected to any one of the intermediate flow path 44, the first sample introduction path 34a, the second sample introduction path 34b, and the introduction path 32.

The present invention is not limited to the embodiment described above and may be modified in various manners without departing from the gist of the present invention. In the embodiment described above, the cell culture system in which the sampling system 10 and the cell culture device 200 are configured separately is illustrated, but the cell culture system may be one in which the sampling system 10 and the cell culture device 200 are integrated.

The above embodiment is summarized as follows.

The above embodiment discloses a sampling method using a sampling system (10) including a sampling channel (30) that collects a liquid sample of a cell culture device (200), a biosensor (28) and a gas concentration sensor (62) being provided in the sampling channel so as to be in contact with the sample, the sampling method including: a sampling step of circulating the sample from the cell culture device to the sampling channel and measuring concentrations of predetermined components in the sample by the biosensor and the gas concentration sensor; a cleaning step of circulating a cleaning liquid through the sampling channel to circulate the cleaning liquid through the biosensor and the gas concentration sensor after the sampling step; and an air introduction step of replacing the cleaning liquid remaining inside the gas concentration sensor with air after the cleaning step, in which the sampling step is performed two or more times, and the second and subsequent sampling steps are performed after the air introduction step.

In the above sampling method, the sampling system may include: an introduction path (32) that introduces the cleaning liquid to an upstream side of the biosensor in the sampling channel; an air introduction path (35) that is connected to an upstream side of the gas concentration sensor in the sampling channel and is opened to the atmosphere; a first opening/closing portion (72a) that opens and closes an upstream side of a connection portion (38) with the introduction path in the sampling channel; a second opening/closing portion (72d) that opens and closes the introduction path; a third opening/closing portion (72f) that opens and closes the air introduction path; and a pump (74) provided in the sampling channel, and in the air introduction step, the air may be guided from the air introduction path to the sampling channel by driving the pump in a state where the first opening/closing portion and the second opening/closing portion are closed and the third opening/closing portion is opened.

In the above sampling method, a sterile filter (52) may be provided in the air introduction path, and in the air introduction step, the air that has passed through the sterile filter may be guided to the sampling channel.

In the above sampling method, the sampling system may include an introduction path that introduces the cleaning liquid to an upstream side of the biosensor in the sampling channel, the sampling channel includes a sample flow path (36) provided with the biosensor and the gas concentration sensor and forming a downstream side of a connection portion with the introduction path in the sampling channel, a first sample introduction path (34a) that guides a first sample of a first cell culture device (200A) that is the cell culture device to the connection portion, and a second sample introduction path (34b) that guides a second sample of a second cell culture device (200B) to an upstream side of the biosensor and the gas concentration sensor in the sample flow path, and the sampling step may include a first sample introduction step of introducing the first sample of the first cell culture device from the first sample introduction path into the sample flow path; and a second sample introduction step of introducing the second sample of the second cell culture device from the second sample introduction path into the sample flow path.
FIG. 1
   - 16: CONTROLLER
   - 18: CLEANING LIQUID STORAGE PORTION
   - 20: STANDARD SOLUTION STORAGE PORTION
   - 22: WASTE LIQUID STORAGE PORTION
   - 60: PH SENSOR
   - 64: O₂ SENSOR
   - 66: CO₂ SENSOR
   - 68: GLUCOSE SENSOR
   - 70: LACTIC ACID SENSOR
   - 200: (200A) FIRST CELL CULTURE DEVICE
   - 200: (200B) SECOND CELL CULTURE DEVICE
FIG. 3
   - S1: PREPARATION STEP
   - S2: PRIMING STEP
   - S3: AIR INTRODUCTION STEP
   - S4: SAMPLING STEP
   - S8: CELL CULTURE IS COMPLETED
   - S9: CLEANING STEP
   - S10: CALIBRATION STEP
FIG. 4
   SAMPLING STEP
   S5 SELECT SAMPLE TO BE COLLECTED
   S6 FIRST SAMPLE INTRODUCTION STEP
   S7 SECOND SAMPLE INTRODUCTION STEP
   FIRST SAMPLE
   SECOND SAMPLE
FIG. 5
   - 16: CONTROLLER
   - 18: CLEANING LIQUID STORAGE PORTION
   - 20: STANDARD SOLUTION STORAGE PORTION
   - 22: WASTE LIQUID STORAGE PORTION
   - 60: PH SENSOR
   - 64: O₂ SENSOR
   - 66: CO₂ SENSOR
   - 68: GLUCOSE SENSOR
   - 70: LACTIC ACID SENSOR
   - 200: (200A) FIRST CELL CULTURE DEVICE
   - 200: (200B) SECOND CELL CULTURE DEVICE
   - CLAMP: OPEN
   - CLAMP: CLOSED
FIG. 6
   - 16: CONTROLLER
   - 18: CLEANING LIQUID STORAGE PORTION
   - 20: STANDARD SOLUTION STORAGE PORTION
   - 22: WASTE LIQUID STORAGE PORTION
   - 60: PH SENSOR
   - 64: O₂ SENSOR
   - 66: CO₂ SENSOR
   - 68: GLUCOSE SENSOR
   - 70: LACTIC ACID SENSOR
   - 200: (200A) FIRST CELL CULTURE DEVICE
   - 200: (200B) SECOND CELL CULTURE DEVICE
   - CLAMP: OPEN
   - CLAMP: CLOSED
FIG. 7
   - 16: CONTROLLER
   - 18: CLEANING LIQUID STORAGE PORTION
   - 20: STANDARD SOLUTION STORAGE PORTION
   - 22: WASTE LIQUID STORAGE PORTION
   - 60: PH SENSOR
   - 64: O₂ SENSOR
   - 66: CO₂ SENSOR
   - 68: GLUCOSE SENSOR
   - 70: LACTIC ACID SENSOR
   - 200: (200A) FIRST CELL CULTURE DEVICE
   - 200: (200B) SECOND CELL CULTURE DEVICE
   - CLAMP: OPEN
   - CLAMP: CLOSED
FIG. 8
   - 16: CONTROLLER
   - 18: CLEANING LIQUID STORAGE PORTION
   - 20: STANDARD SOLUTION STORAGE PORTION
   - 22: WASTE LIQUID STORAGE PORTION
   - 60: PH SENSOR
   - 64: O₂ SENSOR
   - 66: CO₂ SENSOR
   - 68: GLUCOSE SENSOR
   - 70: LACTIC ACID SENSOR
   - 200: (200A) FIRST CELL CULTURE DEVICE
   - 200: (200B) SECOND CELL CULTURE DEVICE
   - CLAMP: OPEN
   - CLAMP: CLOSED
FIG. 9
   - 16: CONTROLLER
   - 18: CLEANING LIQUID STORAGE PORTION
   - 20: STANDARD SOLUTION STORAGE PORTION
   - 22: WASTE LIQUID STORAGE PORTION
   - 60: PH SENSOR
   - 64: O₂ SENSOR
   - 66: CO₂ SENSOR
   - 68: GLUCOSE SENSOR
   - 70: LACTIC ACID SENSOR
   - 200: (200A) FIRST CELL CULTURE DEVICE
   - 200: (200B) SECOND CELL CULTURE DEVICE
   - CLAMP: OPEN
   - CLAMP: CLOSED

## Claims

1. A sampling method using a sampling system including a sampling channel that collects a liquid sample of a cell culture device,
a biosensor and a gas concentration sensor being provided in the sampling channel so as to be in contact with the sample,
the sampling method comprising:
a sampling step of circulating the sample from the cell culture device to the sampling channel and measuring concentrations of predetermined components in the sample by the biosensor and the gas concentration sensor;
a cleaning step of circulating a cleaning liquid through the sampling channel to circulate the cleaning liquid through the biosensor and the gas concentration sensor after the sampling step; and
an air introduction step of replacing the cleaning liquid remaining in the gas concentration sensor with air after the cleaning step,
wherein the sampling step is performed two or more times, and
the second and subsequent sampling steps are performed after the air introduction step.

2. The sampling method according to claim 1,
wherein the sampling system includes:
an introduction path that introduces the cleaning liquid to an upstream side of the biosensor in the sampling channel;
an air introduction path connected to an upstream side of the gas concentration sensor in the sampling channel and opened to an atmosphere;
a first opening/closing portion that opens and closes an upstream side of a connection portion with the introduction path in the sampling channel;
a second opening/closing portion that opens and closes the introduction path;
a third opening/closing portion that opens and closes the air introduction path; and
a pump provided in the sampling channel, and
in the air introduction step, the air is guided from the air introduction path to the sampling channel by driving the pump in a state where the first opening/closing portion and the second opening/closing portion are closed and the third opening/closing portion is opened.

3. The sampling method according to claim 2,
wherein the air introduction path is provided with a sterile filter, and
in the air introduction step, the air that has passed through the sterile filter is guided to the sampling channel.

4. The sampling method according to claim 1, wherein
the sampling system includes an introduction path that introduces the cleaning liquid to an upstream side of the biosensor in the sampling channel, and
the sampling channel includes:
a sample flow path provided with the biosensor and the gas concentration sensor and forming a downstream side of a connection portion with the introduction path in the sampling channel;
a first sample introduction path that guides a first sample of a first cell culture device that is the cell culture device to the connection portion; and
a second sample introduction path that guides a second sample of a second cell culture device to an upstream side of the biosensor and the gas concentration sensor in the sample flow path, and
the sampling step includes:
a first sample introduction step of introducing the first sample of the first cell culture device from the first sample introduction path into the sample flow path; and
a second sample introduction step of introducing the second sample of the second cell culture device from the second sample introduction path into the sample flow path.
